(19) Europäisches Patentamt / European Patent Office / Office européen des brevets

(11) **EP 4 420 703 B1**

(12) **EUROPEAN PATENT SPECIFICATION**

(45) Date of publication and mention
of the grant of the patent:
**31.12.2025 Bulletin 2026/01**

(21) Application number: **24165464.9**

(22) Date of filing: **12.04.2019**

(51) International Patent Classification (IPC):
**A61M 16/06** (2006.01)

(52) Cooperative Patent Classification (CPC):
**A61M 16/0683;** A61M 16/0633; A61M 16/1075;
A61M 16/16; A61M 2205/0216; A61M 2207/00

(54) **HEADGEAR FOR USE WITH A PATIENT INTERFACE OF A RESPIRATORY THERAPY SYSTEM**

KOPFBEDECKUNG ZUR VERWENDUNG MIT EINER PATIENTENSCHNITTSTELLE EINES ATEMTHERAPIESYSTEMS

CASQUE DESTINÉ À ÊTRE UTILISÉ AVEC UNE INTERFACE PATIENT D'UN SYSTÈME DE THÉRAPIE RESPIRATOIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.04.2018 US 201862657427 P**

(43) Date of publication of application:
**28.08.2024 Bulletin 2024/35**

(62) Document number(s) of the earlier application(s) in accordance with Art. 76 EPC:
**19785042.3 / 3 773 845**

(73) Proprietor: **Fisher & Paykel Healthcare Limited 2013 Auckland (NZ)**

(72) Inventor: **DUTHIE, Neil Gray 2013 Auckland (NZ)**

(74) Representative: **Dehns 10 Old Bailey London EC4M 7NG (GB)**

(56) References cited:
WO-A1-2004/041341    WO-A1-2006/000046
WO-A1-2006/072128    WO-A1-2014/165906
WO-A1-2015/043119    WO-A1-2018/065926
US-A1- 2012 145 157

## Description

## BACKGROUND OF THE DISCLOSURE

### Field of the Disclosure

The present invention generally relates to headgear for use with a patient interface of a respiratory therapy system.

### Description of the Related Art

One example of use of a respiratory therapy system is for the treatment of obstructive sleep apnea (OSA) by continuous positive airway pressure (CPAP) flow generator systems involving the continuous delivery of pressurized gases to the air-ways of a human via a breathing gas delivery conduit and a patient interface. Such a patient interface may be any one of the following:

- a nasal interface configured to seal around the nares or nose of the patient;

- an oral mask configured to seal around the mouth of the patient; or

- a full-face mask configured to seal around both the mouth and nose of the patient.

Headgear is typically used to secure the patient interface on the user's head. The headgear typically comprises one or more straps and/or panels that are secured to the patient interface and which pass around one or more parts of the patient's head. The headgear is typically configured to provide one or more of following functions:

a) to locate the patient interface in the desired position on the user's face.

b) to maintain the seal between the patient interface and the user's face by application of compression forces between the seal of the patient interface and the user's face.

c) to distribute the required forces between the patient interface and the user.

d) to improve the comfort of the patient during therapy.

e) to improve the appearance of the product. Whilst the appearance might be considered insubstantive, it has been shown that the aesthetics of components of a respiratory therapy system can affect how willing the user is to use the system and thus ultimately affect the success of the therapy.

f) to be cost effective to manufacture, and in particular to minimise material usage. This can be a particular concern for products that are disposable, or at least which are used for a relatively short period, such as less than one month for example. One example of this is in a hospital setting where patients are typically prescribed non-invasive ventilation (NIV) for relatively short periods of time in comparison to home treatments in which the patient may use the mask continuously for a longer period, such as up to six months, before replacing it. CPAP headgear may be washed and reused whereas typically NIV headgear is single use and is then disposed of.

Such headgear can comprise a number of different sizes and configurations of straps that are integrally formed, or joined together. Further achieving one or more of the above functions can be difficult, as one function can conflict with another.

An example of known headgear is that provided with the Simplus ® or Nivairo ® product of Fisher & Paykel Healthcare Limited. That particular product uses headgear comprising a pair of upper and lower side straps which are configured to be removably secured to respective parts of the patient interface. The upper and lower side straps extend along the sides of the user's head and terminate at a rear panel that engages the rear of the patient's head. A crown strap is further provided, which extends over the crown of the user's head from the upper side straps. US2012/145157A1 discloses an example of headgear.

## SUMMARY

The present disclosure stems from work undertaken towards providing improved headgear to better fulfil any one or more of the above functions.

It is an object of the present disclosure to provide an improved headgear for use with a patient interface, and/or that will at least provide the public or the medical profession with a useful choice.

In one aspect of the invention, headgear configured to be secured to a patient interface of a respiratory therapy system, to mount the patient interface on a user's head, is provided by claim 1, the headgear comprising:

a pair of side straps, each side strap having first and second opposed ends, a first end of each strap being secured to, or being configured to be secured to, the patient interface and to extend along the sides of the user's head;

a rear panel configured to engage the rear of the user's head, the second end of each side strap being joined to the rear panel such that the pair of side straps extend from the rear panel; wherein

the rear panel comprises a main body and a pair of laterally extending arms, the second end of each side strap being joined to a respective arm;

the main body comprising:

opposed top and base margins which are spaced apart by a distance,

substantially straight, outwardly inclined, opposed side margins extending between the top and base margins, and

a central longitudinal axis bisecting the top and base margins;

wherein the area of the main body is defined by the top and base margins and the side margins, including the area defined where the top margin notionally extends towards the laterally extending arms and intersects with where the side margins notionally extend towards the laterally extending arms;

each laterally extending arm being inclined relative to the central longitudinal axis, extending away from a respective side margin of the main body and comprising a distal arm end,

each distal arm end having a width measured in a direction substantially parallel with the central longitudinal axis;

wherein wherein the ratio of the area of both lateral extending arms to the main body is between 0.1 and 0.3.

The total area of the rear panel may be between 6000 and 13000mm$^2$.

The ratio of the area of the main body to the total area of the rear panel may be between 0.7 and less than 1.0.

The laterally extending arms may intersect the main body above a vertical midpoint of the main body.

Optionally, when the length of the top margin of the main body is divided into two lateral thirds and a central third, each laterally extending arm can be said to intersect the top margin of the main body within the lateral third proximal to the laterally extending arm in question.

The entire rear panel may be constrained within a rectangle R of less than 200mm by 100mm.

The side margins of the main body of the rear panel may be at least partially substantially parallel and substantially equal length such that the main body is substantially rectangular.

The side margins of the main body of the rear panel may be of substantially equal length and may be at least partially inclined relative to the base margin such that the main body is substantially trapezoidal.

In some examples two pairs of side straps may be provided, one pair comprising upper side straps, the other pair comprising lower side straps.

Each lateral arm may be of substantially uniform width along its length.

Each distal arm end may comprise a substantially straight end margin. The end margin may be parallel with the longitudinal axis. The end margin may be inclined relative to the longitudinal axis. The end margin may be inclined relative to the longitudinal axis at an angle between 0.1 and 20°.

The side margins of the rear panel may be inclined relative to the longitudinal axis at a different angle to the angle of inclination of the lateral arms relative to the longitudinal axis.

The side margins of the rear panel may be inclined at an angle between 0° and 40° relative to the longitudinal axis of the rear panel.

The longitudinal axis of each arm may be inclined at an angle between 40° and 90° relative to the longitudinal axis of the rear panel.

The entire rear panel may be constrained within a notional rectangle of 100-300mm wide by 50-120, tall, and in some embodiments within a notional rectangle of 180-200mm by 75-95mm.

The rear panel may be defined by any one or more of the following ratios:

a. the ratio of the length of a lateral arm to the length of the main portion is between 0.65 and 0.8, length being measured in a direction perpendicular to the longitudinal axis;

b. the ratio of the height of a lateral arm to the height of the main portion, height being measured in a direction parallel to the longitudinal axis, is between 1.1 and 1.6;

c. the ratio of the length of the entire rear panel to the length of the main portion is between 2.0 and 3.0;

d. the ratio of the area of a lateral arm to the area of the main portion is between 0.3 and 0.5;

e. the ratio of the area of both lateral arms to the area of the entire rear panel is between 0.3 and 0.7.

f. the ratio of a cut out or recess formed adjacent the upper margin of the rear panel as defined by the area between the upper margin of the rear panel and a notional horizontal line extending between and connecting the two most upper points of the lateral arms, to the area of the entire rear panel is between 0.3 and 0.5.

g. the rear panel comprises between 30 and 70% of a notional rectangular area that surrounds and contacts all extreme points of the rear panel.

The rear panel may be defined by any one or more of the following ratios:

a. the total area of the rear panel is between 8000 and 13000mm$^2$;

b. the ratio of the area of the main portion to the entire area of the main panel is between 0.75 and 1.0.

The rear panel may be defined by the following formula:

$$w2 = 1/2\ d1\ -\ 1/2\ c;$$

where w2 = the width of the lateral arm as measured in a direction substantially parallel with the longitudinal axis, d1 = the distance between opposed top and base margins of the main portion, and c = the clearance between adjacent rear panels required during manufacture of multiple rear panels on a sheet of material to ensure a satisfactory quality of cut.

Each inclined arm may extend from a respective side margin and the top margin of the main body.

The main body of the rear panel may comprise opposing top and base margins, opposing side margins connecting the opposing top and base margins, a central vertical plane connecting the mid points of the opposing top and base margins, and a central horizontal plane connecting the mid points of the opposing side margins,

wherein the opposing side margins are inclined relative to the opposing top and base margin, and are of equal length, such that the main body is substantially trapezoidal;

wherein each inclined arm extends from one of the pair of side margins and the top margin of the trapezoidal shaped main body above the central horizontal plane and extend away from both the central horizontal and vertical planes, and at an angle between 0° and 90° from the horizontal plane.

Each angled arm may extend away from one of the pair of side margins and the top margin of the trapezoidal shaped main body, and the area of each angled arm is less than 15% of the area of the main body.

Each angled arm may extend from one of the pair of side margins and the top margin of the main body, and each upper side strap connects entirely to a respective one of the pair of angled arms and the at least one of the pair of lower straps connects entirely to the main body.

Each laterally extending arm may taper, flare or remain substantially constant along its length.

The distal arm end of each laterally extending arm may be stepped or tapered or waisted, so that the distal arm end is of narrower width than the remainder of the laterally extending arm.

The ratio of the width of each laterally extending arm to the width of the main body portion may be between 0.4:1 and 0.8:1.

Another aspect of this disclosure may be said to broadly consist in a patient interface assembly comprising;

a patient interface, and

the headgear of any one of the previous aspects or embodiments.

A further aspect of this disclosure may be said to broadly consist in a respiratory therapy system comprising the headgear of any one of the above statements and any one or more of:

a. a breathing gas flow generator;

b. a breathing gas humidifier;

c. a gas delivery conduit, which may or may not be heated; and/or

d. a patient interface.

Further aspects of the disclosure, which should be considered in all its novel aspects, will become apparent from the following description.

## DESCRIPTION OF THE DRAWINGS

A number of embodiments of the disclosure will now be described by way of example with reference to the drawings in which:

**Figure 1** is a perspective view from the rear of a headgear in accordance with the present disclosure;

**Figure 2** is a perspective view from the rear and side of the headgear of Figure 1;

**Figure 3** is a rear view of the headgear of Figures 1 and 2;

**Figure 4** is a plan view of the exterior of the headgear of Figures 1 to 3 when laid flat;

**Figure 5** is a plan view of the interior of the headgear of Figures 1 to 4 when laid flat;

**Figures 6 to 9** are plan views of a rear panel of the headgear of Figures 1 to 5, showing different dimensions and aspects of the rear panel;

**Figure 10** is a lay flat view of a continuous sheet of textile in an industry standard width of 59" (1498mm) with an example of the nesting patterns and yield that can be achieved with the rear panel of Figures 6 to 9;

**Figure 11** is a hypothetical nesting pattern of two rear panels of headgear in accordance with the current disclosure;

**Figure 12** is an enlarged view corresponding to Figure 10;

**Figures 13 to 15** are lay flat views of a continuous sheet of textile in an industry standard width of 59" (1498mm) with an example of other nesting patterns and yield that can be achieved with the rear panel of headgear in accordance with the current disclosure;

**Figures 16 to 19** are plan views of a rear panel of headgear in accordance with the current disclosure showing different dimensions and aspects of the rear panel;

**Figures 20 to 25** are plan views of another embodiment of a rear panel of the headgear of Figures 1 to 5, showing different dimensions and aspects of the rear panel with Figure 22 being an enlarged view of Area A of Figure 24; and

**Figures 26 to 31** are plan views of a further embodiment of a rear panel of the headgear of Figures 1 to 5, showing different dimensions and aspects of the rear panel, with Figure 28 being an enlarged view of Area A of Figure 29j.

## DETAILED DESCRIPTION

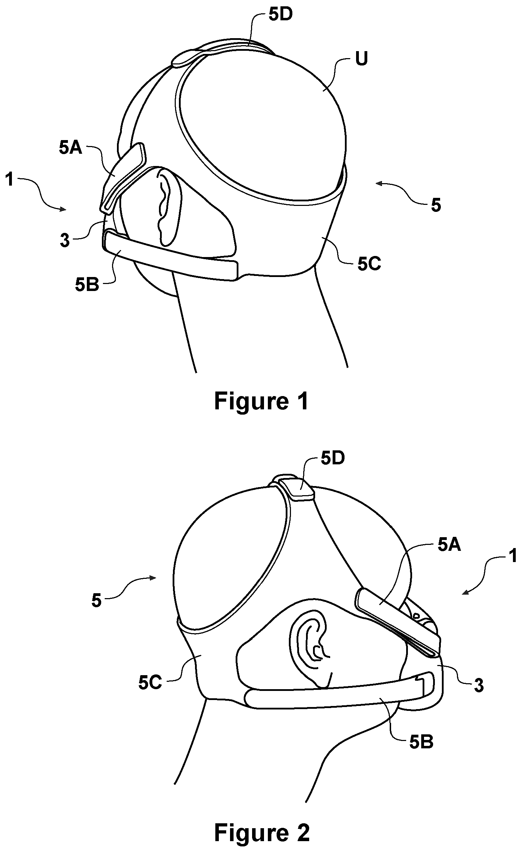

With reference initially to Figures 1 to 3, an embodiment of a patient interface assembly 1 comprising a patient interface 3 and headgear 5 is illustrated on a user U, the patient interface in this example being a full face mask covering both the nose and mouth of the user U. The patient interface assembly 1 can be used in the field of respiratory therapy and therefore in any respiratory treatment, respiratory assistance, resuscitation or ventilation system. In some embodiments, the interface assembly 1 has particular utility with forms of positive pressure respiratory therapy. For example, the interface assembly 1 can be used for administering continuous positive airway pressure ("CPAP") treatments, variable positive airway pressure ("VPAP") treatments and/or bi-level positive airway pressure ("BiPAP") treatments. The interface assembly 1 can be compatible with one or more different types of suitable CPAP or non-invasive ventilation (NIV) systems.

The patient interface 3 can comprise any of a plurality of different types of suitable mask configurations. For example, certain features, aspects and advantages of the present disclosure can be utilized with nasal masks, full face masks, oronasal masks, total face, or any other positive pressure mask. Although the illustrated mask is a full face mask, the scope of the present disclosure should not be limited by the particular embodiments described.

In the illustrated configuration, the patient interface 3 comprises a mask body, optionally a mask frame and a connection port assembly. The mask body is configured to cover the user's mouth and/or nose to deliver respiratory gases to the user. The mask body can be secured to the mask frame 5. The patient interface 3 is held in place on the user U by the headgear 5 that wraps around a part or parts of the user's head. The connection port assembly can be connected to the mask body and/or mask frame, preferably with a releasable connection. In some configurations, the connection port assembly comprises an elbow connector configured to be connected between the mask body and/or mask frame and a gas delivery conduit (not shown).

The mask frame can couple to the mask body and help stabilize the interface 3 on the user's face. The mask frame can be any shape and size to functionally secure the interface 3 to the user's face. The mask frame can be attached to the mask body with interlocking clips, tabs or other functional couplers or may be permanently attached during the assembly process. In this latter case the mask body and mask frame are integral and configured to form a single component. The mask frame can be rigid, substantially rigid or semi-rigid to provide support for the mask body. For example, the mask frame can be at least partially made of a metal or rigid plastic, such as acrylic, polycarbonate or high-density polyethylene.

The mask frame can extend to the user's forehead and may optionally include a forehead rest. The forehead rest, if provided, can help stabilize the interface 1 to the user's face by providing a support point for the interface 1 and connection points for the headgear 5.

If provided, the forehead rest can be a separate flexible piece that is attached or overmoulded onto the mask frame. For example, the forehead rest can be made of a flexible silicone that is overmoulded onto the frame bridge. The flexible material advantageously conforms to the user's forehead anatomy and helps improve comfort to the user with soft material contact. In some configurations, the forehead rest can be attached or integrally formed as part of the mask frame and can be made of the same material as the mask frame 5 and frame bridge.

In this example headgear 5 comprises a pair of upper side straps 5A extending from laterally spaced upper connections on the mask body or mask frame, a pair of lower side straps 5B extending from laterally spaced lower connections on the mask body or mask frame, a rear panel 5C which joins the upper and lower side straps 5A, 5B, at the rear of the head of the user U, and a crown strap 5D which extends over the crown of the head of the user U, from each upper side strap 5A. Suitable connectors, such as incorporating hook and loop fasteners, may be provided to secure the headgear 5 to the mask body or mask frame. In this example, both pairs of upper and lower straps 5A, 5B comprise hook and loop fasteners 6 at their respective strap ends, each strap being looped through the mask frame and/or forehead rest and back on itself. The amount by which each strap 5A, 5B is looped back on itself can provide some adjustment of the size and fit of the headgear 5. Both pairs of upper and lower straps 5A, 5B are further provided with gripping formations 8 at their distal ends.

Any or all of the above straps 5A. 5B, 5C may include adjusters, such as buckles, configured to enable the length of the strap(s) to be adjusted.

Any one or more of the straps 5A. 5B, 5C and/or rear panel 5C may be elastic or inelastic or comprise portions that are elastic or inelastic. Any one or more the straps 5A. 5B, 5C or rear panel 5C may comprise more rigid portions, for example, to help maintain a desired shape of the headgear 5. The materials, construction, and elasticity may vary between straps 5A. 5B, 5C and/or rear panel 5C however, the headgear 5 alternatively may be of uniform construction with the same material and elasticity used throughout the headgear. This may further reduce costs by enabling the entire assembly to be cut from sheets of the same material.

In this example crown strap 5C comprises two crown strap portions 5DA, 5DB that are releasably connected together via the end of one crown strap portion 5DA being received in an aperture 21 of the other crown strap portion 5DB. The end of first crown strap portion 5DA is waisted such that there is a narrower width portion 23 spaced from the end of the first crown strap portion 5DA. The waisted portion 23 is substantially the same width as the aperture 21 and is located in the aperture 21 when the two crown strap portions 5DA, 5DB are connected together, with the wider end 24 of first crown strap portion 5DA protruding through the aperture and resisting removal of the first crown strap portion 5DA through the aperture 21.

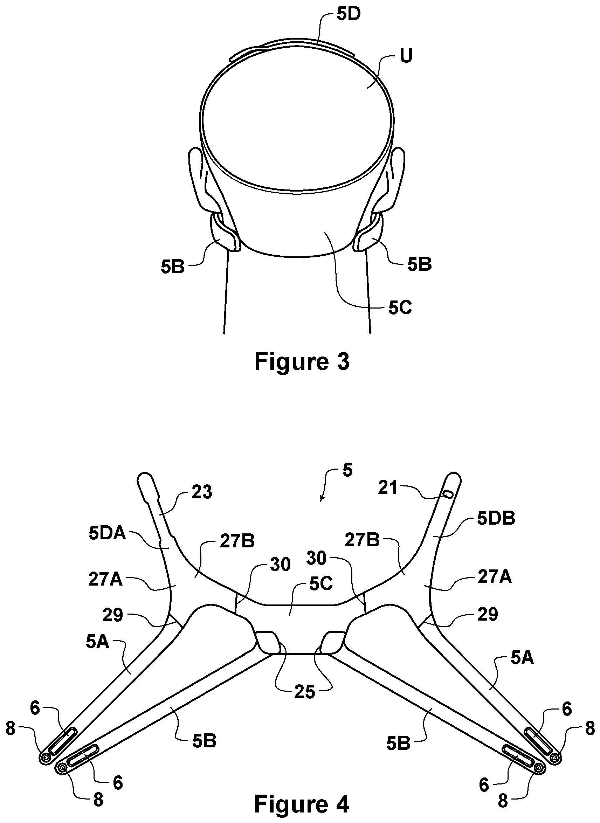

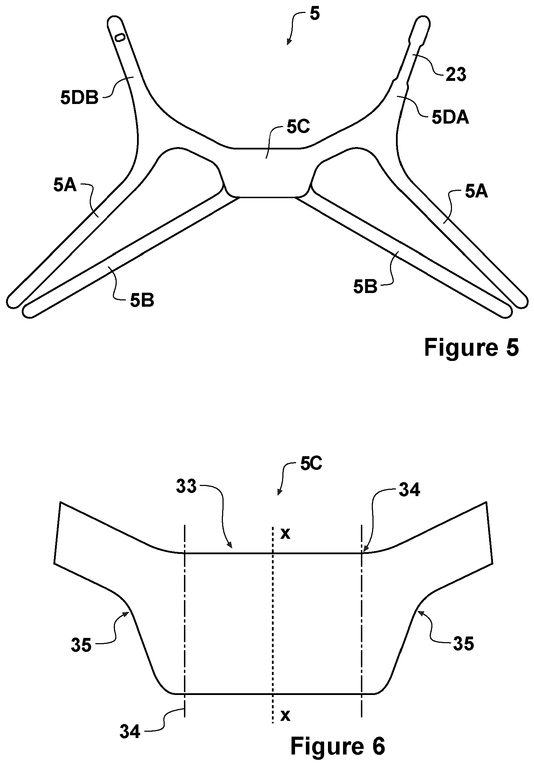

With reference additionally to Figures 4 and 5, the margins of the rear panel 5C are shown, with the lower side straps 5B being joined to the rear panel 5C at joins 25. The inner end of each crown strap portion 5DA, 5DB comprises a bifurcated region 27 forming two legs 27A, 27B. The upper side straps 5A are joined to respective legs 27A at joins 29 whilst legs 27B are joined 30 to respective upper margins 31 of the rear panel 5C. The joins may use any suitable joining means which can include any one or more of stitching, gluing, ultra-sonic welding, RF welding or any other form of seam welding.

The upper and lower side straps 5A, 5B are joined to the rear panel 5C such that the upper straps 5A are non-parallel with the lower side straps 5B such that the distal ends of the upper and lower side straps 5A, 5B on each side of the rear panel 5C are inclined toward one another. The angle between the upper and lower side straps 5A, 5B can be varied depending on the connection of the upper and lower side straps 5A, 5B to the patient interface.

Referring additionally to Figure 6, the rear panel 5C can be considered to comprise a main portion or body 33 and two opposed lateral portions or arms 35, with a notional transition line 34 between the main and lateral portions 33, 35. In this example, the main portion 33 can be considered to be rectangular. The two lateral portions 35 each extend outwardly from the central plane or longitudinal axis XX and are inclined upwardly away from the main portion 33 and away from the longitudinal axis to facilitate connection with the upper straps 5A and locate those straps 5A in the correct position on the back of the user's head.

Each upper strap 5A is connected entirely to a respective one of the lateral portions 35 of the rear panel 5C in an upper corner region of the rear panel 5C. Each lower strap 5B is connected to a lower corner area of the rear panel 5C, that includes at least a portion of the lateral portion 35 and main portion 33.

The boundaries of the main portion 33 are defined by a centrally located rectangle with upper and lower limits formed by the substantially straight parallel regions of the upper and lower margins of the rear panel 5C and lateral limits formed by notional vertical lines 34 the ends of which are located at points where the upper margin transitions from substantially horizontal by a line of best fit, to a curved line into the lateral portions 35. By 'substantially horizontal', we include that the upper margin is slightly curved.

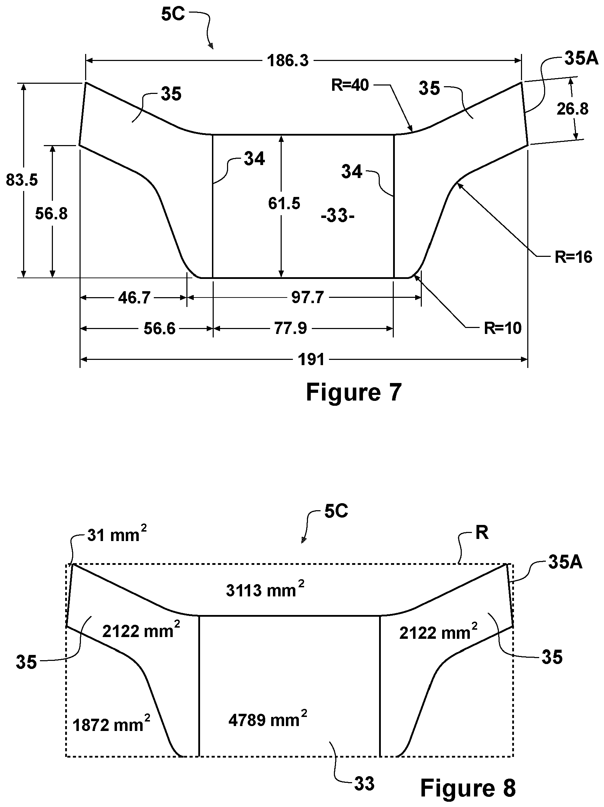

Referring to Figure 7, example relative dimensions (mm) of the rear panel 5C are:

- The main portion 33 is defined by a rectangle with dimensions of less than 100mm by 70mm, and in one example 77.9mm by 61.5mm.
- The lateral portions 35 are constrained within a rectangle of less than 60mm by 90mm, and in one example 56.6mm by 83.5 mm.
- The entire rear panel 5C is constrained within a rectangle R, shown in dashed line, of less than 200mm by 100mm, and in one example 191mm by 83.5mm.
- The ratio of the area of each lateral portion 35 to the area of the main portion 33 is between 0.8 and 0.9 and preferably 0.886.

Referring to Figure 8 the rear panel 5C can be effectively described with various example ratios relating the lateral portions 35 and main portion 33. Example ratios are listed below.

- The ratio of the width of a lateral portion 35 to the width of the main portion 33 may be between 0.7 and 0.8, and in one example is 0.73.
- The ratio of the height of a lateral portion 35 to the height of the main portion 33 may be between 1.2 and 1.5, and in one example is 1.36.
- The ratio of the width of the entire rear panel 5C to the width of the main portion 33 may be between 2.0 and 3.0, and in one example is 2.45.
- The ratio of the area of a lateral portion 35 to the area of the main portion 33 is may be between 0.4 and 0.5, and in one example 0.443.
- The ratio of the area of both lateral portions 35 to the area of the entire rear panel 5C may be between 0.4 and 0.7, and in one example is 0.470.
- The ratio of a cutout formed in the top of the rear panel 5C defined by the area A between the upper margin 41 of the rear panel 5C and a horizontal line 43 connecting the two most upper points 45 of the lateral portions 35 to the area of the entire rear panel 5C may be between 0.3 and 0.5, and in one example is 0.347.
- The rear panel 5C may be between 30 and 70%, and in one example makes up 56.64%, of a notional rectangular area A that extends to touch all extreme points of the rear panel 5C.
- The ratio of the width of the lower margin of the rear panel 5C to the width of the entire rear panel 5C may be between 0.4 and 0.8, and in one example is 0.51.

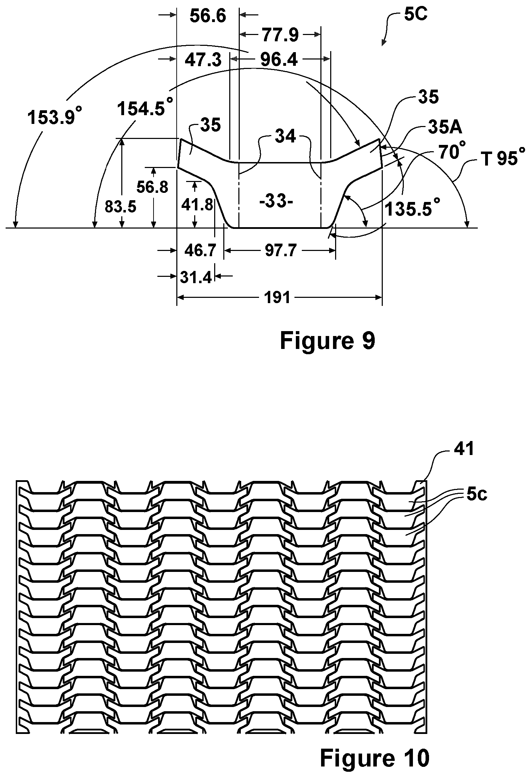

Referring to Figure 9, example dimensions of, and example angles between, the different parts of the rear panel 5C are shown. These include a lateral portion 35 tip angle T which defines the angle of the straight, distal end margin 35A of each lateral portion 35 relative to the horizontal base margin of the main portion 33.

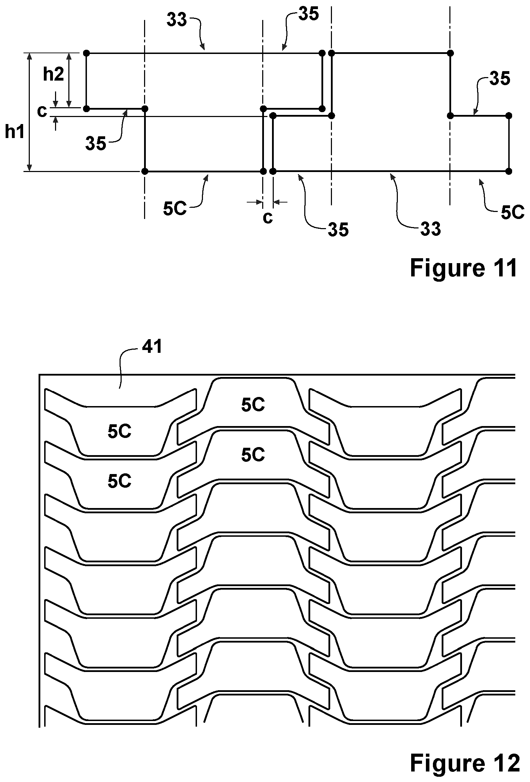

Referring to Figures 10 and 12, a continuous sheet of textile 41 in an industry standard width of 59" (1498mm) is shown with an example of the nesting patterns and yield that can be achieved with a rear panel 5C in accordance with this disclosure. It is desirable to firstly use an industry standard width of material, and to secondly maximise the amount of that material that is used, minimising the amount that is wasted and therefore reducing the overall manufacturing cost of the headgear assembly.

- the shape and configuration of the rear panel 5C is such that a relatively high yield is possible when adjacent panels 5C are placed alongside each other with each intermediate panel 5C in a given row of rear panels 5C being inverted relative to the adjacent panel 5C.
- In this position the angles and shape of the lateral portions 35 are complimentary with each other which allows the lateral portions of each row to be positioned between the lateral portions of the adjacent rows without the rear panels contacting each other. This arrangement allows for a relatively high yield nesting pattern which decreases material waste and reduces the cost of the component due to the disused areas formed between rows of rear panels being inhabited partially/substantially by the adjacent rows of rear panels.
- In this disclosure yields of over 75% are possible, and in this example a yield of 83.9% can be achieved.

Referring to Figure 11, a hypothetical nesting pattern of two rear panels 5D that could achieve the maximum yield possible while leaving a clearance c of 1/8th of an inch between pieces to enable the die cutting blades to effectively operate is shown. This nesting pattern uses the following formula:

$$w2 = 1/2\ h1 - 1/2\ c;$$

where w2 = the width of the lateral arm as measured in a direction substantially parallel with the longitudinal axis, d1 = the distance between opposed upper and base margins of the main portion, and c = the clearance between adjacent rear panels required during manufacture of multiple rear panels on a sheet of material to ensure a satisfactory quality of cut.

The clearance c can be varied in order to achieve a satisfactory cut between adjacent rear panels 5D. It is desirable from a material wastage point of view that this clearance is as small as possible. However, if the clearance equals zero, the quality of the cut margin of each rear panel 5 may be compromised.

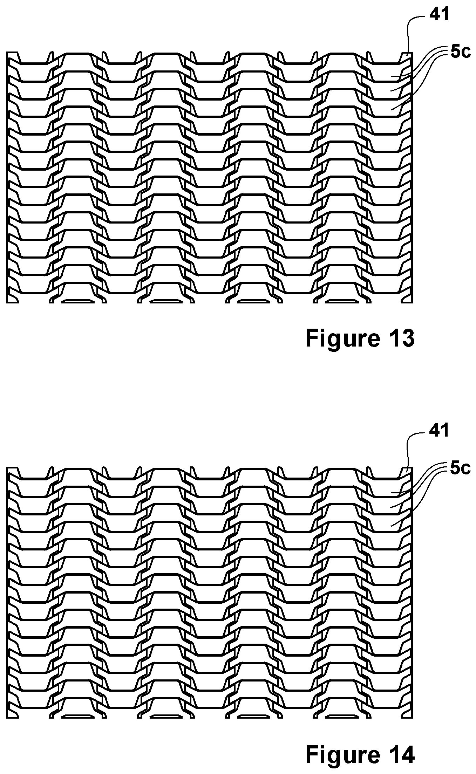

Figures 13 to 15 show different examples of the rear panel 5C where the tip angle T of the of the straight end margin 35B of each lateral portion 35 relative to the horizontal base margin 33A of the main portion 33 is varied, this tip angle T affecting the yield. In each of these examples a yield of over 83% is achieved. Although the tip angle T will affect the available yield, this angle may also affect the aesthetics of the headgear, and/or the best angle to achieve a suitable joint with the upper side strap 5A. As such choosing the best tip angle T may require consideration of any one or more of these factors, and/or some compromise.

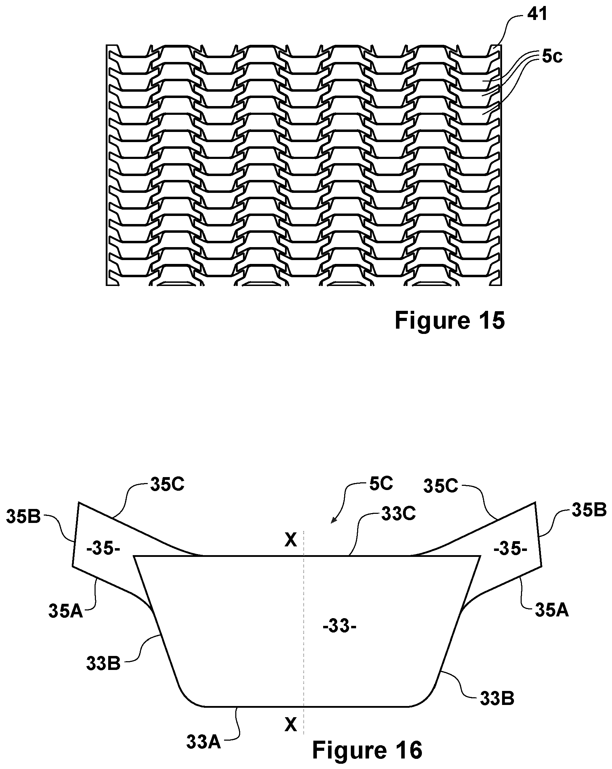

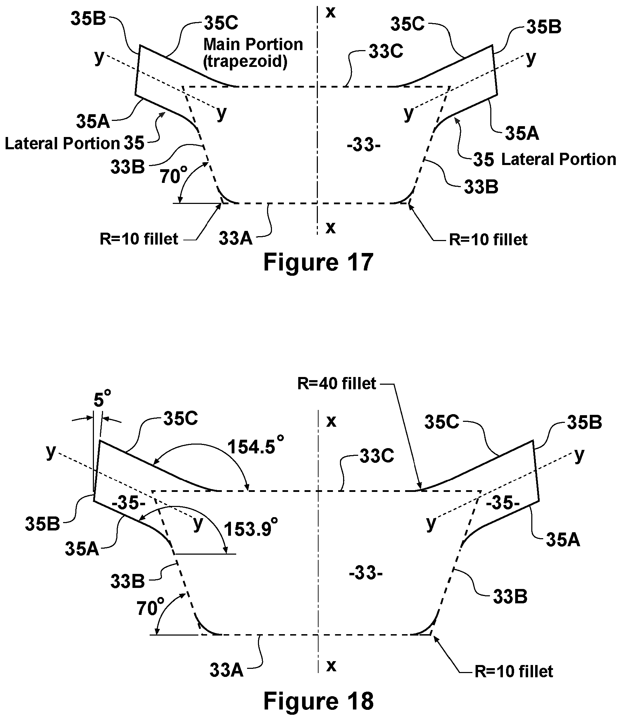

Referring to Figures 16 to 18, the rear panel 5C is considered in an alternative manner as comprising a trapezoidal main portion 33 with two lateral portions 35 each intersecting the trapezoidal main portion 33.

Each lateral portion 35 extends laterally outwardly, and is inclined, away from main portion 33 and from the central vertical plane/longitudinal axis XX such that a longitudinal axis YY of each lateral portion 35 is inclined relative to the longitudinal axis XX and relative to the straight, horizontal upper and lower margins of the main portion 33.

The rear panel 5C may therefore be defined by a main portion or body 33 comprising a substantially straight horizontal lower base margin 33A leading to substantially straight, outwardly inclined side margins 33B. Each side margin 33B leads to a respective lateral portion 35 comprising an elongate outwardly extending arm where the longitudinal axis YY of each arm 35 is inclined relative to the base margin 33A, and the angle of inclination of each arm 35 relative to the base margin 33A is less than the angle of inclination of the side margins 33B. Each lateral arm 35 comprises substantially straight lower, end and upper margins 35A, 35B, 35C, with the end margin 35B being inclined relative to the central longitudinal axis XX of the rear panel 5C. The lower and upper margins 35A, 35C could be parallel, or could taper toward or away from another. The upper margin of each arm 35 leads into the substantially straight upper margin 33C of the panel 5C, the upper margin 33C being parallel with the base margin 33A. The lower margin 35A of each arm 35 leads into a respective side margin 33B of the main body 33.

When considered in this way:

- The rear panel 5C is symmetrical about line XX.
- The straight, horizontal upper and lower margins 33C, 33A of the main portion 33 are parallel.
- The outwardly inclined side margins 33B of the main portion 33, each extend from a separate lateral endpoint of base margin 33A at an angle of 70°, such that side margins 33B extend upwardly and outwardly from the central plane XX.
- The endpoints of side margins 33B intersect the endpoints of top margin 33C such that base margin 33A, side margins 33B and top margin 33C together form a trapezoidal shape which is mirrored about plane XX with top margin 33C > base margin 33A > side margins 33B.
- The intersections of side margins 33B with base margin 33A are filleted with a 10mm radius.
- The two lateral portions 35 extend distally away from the central plane XX at an angle above horizontal.
- Lower arm margin 35A intersects side margin 33B and extends distally away from the central plane XX at an angle of between 15 and 45°, and in one example of 26.1° from a plane parallel to base margin 33A.
- The intersection between lower arm margin 35A and base margin 33A is filleted with a 16mm radius.
- Upper line margin 35C intersects top margin 33C and extends distally away from the central plane XX at an angle of between 15 and 45°, and in one example of 25.5°, from top margin 33C.
- The intersection between upper arm margin 35C and top margin 33C is filleted, in this example with a 40mm radius.
- End arm margin 35B extends from the free end of lower arm margin 35A at an angle of between 0° and 20°, and in this example 5°, from a plane parallel to plane XX such that is extends proximally towards plane XX and intersects the free end of upper arm margin 35C.

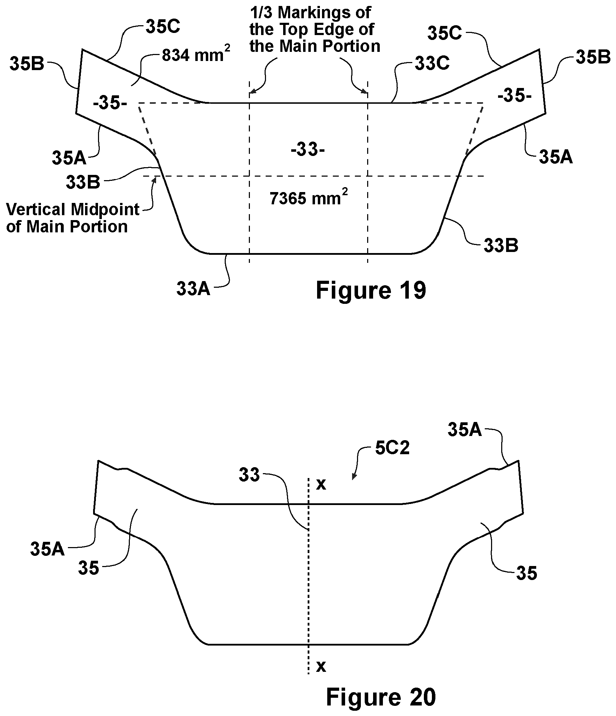

As illustrated in Figure 19:

- The total area of the rear panel 33 may be between 6000 and 13000mm$^2$, and in this example is about 9,000mm$^2$.
- The ratio of the area of both lateral portions 35 to the main portion 33 may be between 0.1 and 0.3, and in this example is about 0.185.
- The ratio of the area of the main portion 33 to the total area of the rear panel 5C may be between 0.7 and 1.0, and in this example is about 0.815.
- The lateral portions 35 intersect the main portion 33 above the vertical midpoint of the main portion.
- With the length of the top edge margin 33C of the main portion 33 divided into two lateral thirds and a central third, each lateral portion 35 can be said to intersect the top edge margin 33C of the main portion 33 within the lateral third proximal to the lateral portion 35 in question.

In this disclosure the main portion or body 33 is defined by the area captured within the top and base margins 33C, 33A and the side margins 33B, including the area defined where the top margin 33C notionally extends towards the lateral portions or arms 35 and intersects with where the side margins 33B notionally extend towards to the lateral portions or arms 35. These notionally extending parts of the top and side margins 33C, 33B are shown in dotted line N in Figure 19 for example.

The headgear 5 may be configured such that the other dimensions and ratios set out above can vary in dependence upon the size of head gear 5 (and rear panel 5A) required. In other words, the rear panel 5A may be configured to be scaled such that the proportions and ratios of various components will remain the same between the smallest to largest size of the rear panel 5A. The panel 5A is configured to be scaleable such that for example the major dimensions of a large size may be 1.5 times bigger than the major dimensions of a small size.

Table 1 below shows example dimensions of three headgear sizes, for headgear 5 in accordance with this disclosure. The headgear rear panel 5C described above is for a size Medium/Large. This is designed to fit a majority of a given set of users. The other two size options show examples of a largest practical rear panel size as well as a smallest practical rear panel size. However, the particular example dimensions listed in the table below are designed to be able to fit the largest and smallest head sizes of which we are aware, and illustrate that variations in proportions between different sizes is possible, i.e. this table lists possible extreme dimensions and as such these three size configurations are not scaled relative to one another as outlined above.

| | Medium / L Rear Panel Dimensions (Adult Patient) | Largest Practical Rear Panel Dimensions (Adult Patient) | Smallest Practical Rear Panel Dimensions (Adult Patient) |
|---|---|---|---|
| Overall Rear Panel Width | **191mm** | **261mm** | **121mm** |
| Central Quadrilateral Region Width | **77.9mm** | **150mm** | **10mm** |
| Overall Rear Panel Height | **83.5mm** | **118.5mm** | **50mm** |

The headgear 5 may provide a relatively low cost solution that is important for use in the NIV environment where masks and associated headgear are disposable and each patient receives a new mask every 14 days typically. The relatively low cost solution is achieved via a unique geometrical shape and configuration that allows relatively high yield manufacturing owing to the efficient nesting pattern. The relatively large single component rear panel 5C may also increase patient comfort and/or improve the aesthetics of the headgear 5, which may help to increase patient compliance.

The headgear 5 may comprise part of a respiratory therapy system which comprises any one or more of:

a. a breathing gas flow generator;

b. a breathing gas humidifier;

c. a gas delivery conduit, which may or may not be heated;

d. a patient interface.

The headgear 5 may comprise part of a patient interface assembly comprising the combination of the headgear 5 and a patient interface 3.

The headgear 5 may be manufactured from any suitable fabric material or combination of materials. Parts of the headgear 5 may for example be manufactured from Breathoprene ®.

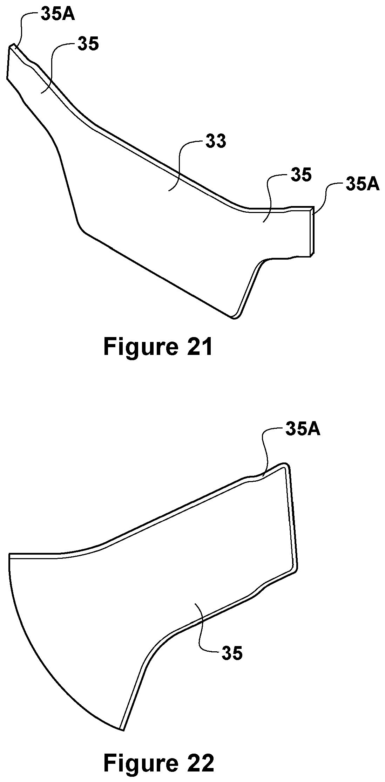

Referring to Figures 20 to 25, another embodiment of rear panel 5C is shown, and will be referred to as rear panel 5C2. Panel 5C2 is closely similar to Panel 5C described above, and like features will be given like references. Only the differences are discussed below.

Rear panel 5C2 can again be considered to comprise a main portion or body 33 and two opposed lateral portions or arms 35, with a notional transition line 37 between the main and lateral portions 33, 35. In this example, the main portion 33 can be considered to be rectangular. The two lateral portions 35 each extend outwardly from the central plane or longitudinal axis XX and are inclined upwardly away from the main portion 33 and away from the longitudinal axis to facilitate connection with the upper straps 5A and locate those straps 5A in the correct position on the back of the user's head. The lateral portions 35 of panel 5C2 terminate in tips 35A of reduced width. These are configured to minimise laminate flare when welded to straps of the headgear.

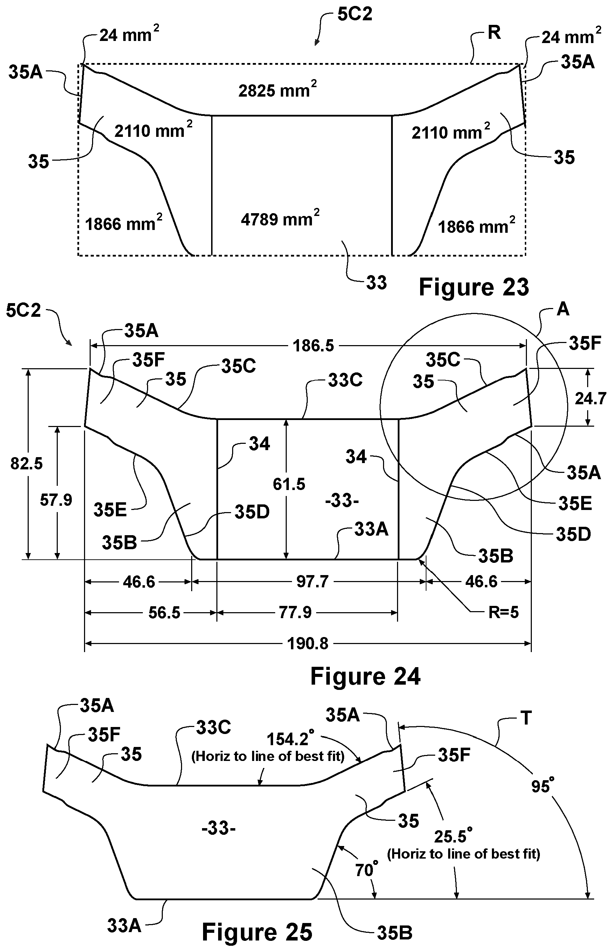

Referring to Figure 24, the example relative dimensions (mm) of the rear panel 5C2 are identical or closely similar to that of panel 5C except that the tip of each lateral arm 35 is approximately 2mm narrower, for the reason discussed above. The radius of curvature between the lower margin of main body 33 and each lateral arm 35 is about 5mm in this example, as compared to about 10mm for panel 5C.

Referring to Figure 24 the rear panel 5C2 can be effectively described with the same example ratios relating the lateral portions 35 and main portion 33, as described above for panel 5C. Example ratios are listed below.

Referring to Figure 25, example dimensions of, and example angles between, the different parts of the rear panel 5C2 are shown. These are substantially the same as those of panel 5C.

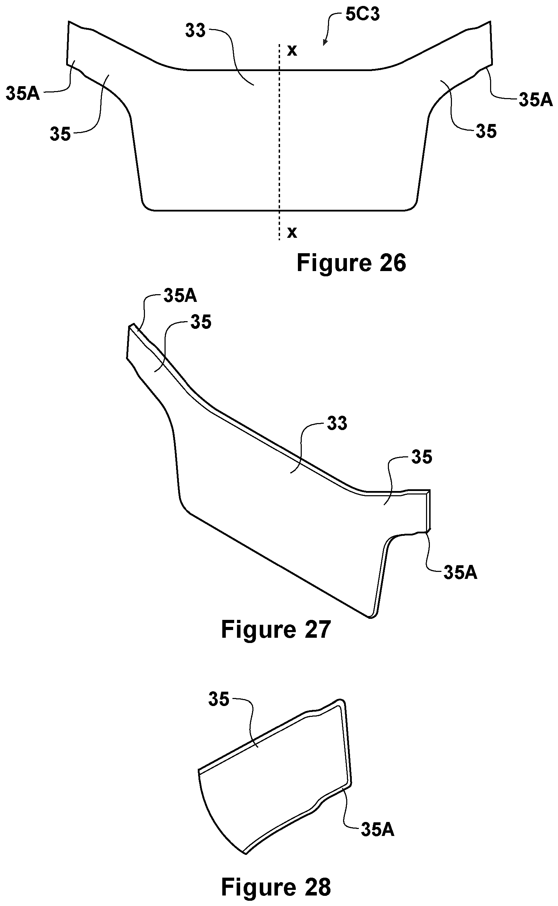

Referring to Figures 26 to 31, another embodiment of rear panel 5C is shown, and will be referred to as rear panel 5C3. Panel 5C3 is closely similar to panels 5C and 5C2 described at length above, and like features will be given like references. Panel 5C3 is a very similar height to panels 5C and 5C2, but is narrower overall, with a wider main portion 33, narrower width lateral portions 35, and lateral portions 35 that are more steeply inclined relative to the horizontal (of the base margin of main portion 33).

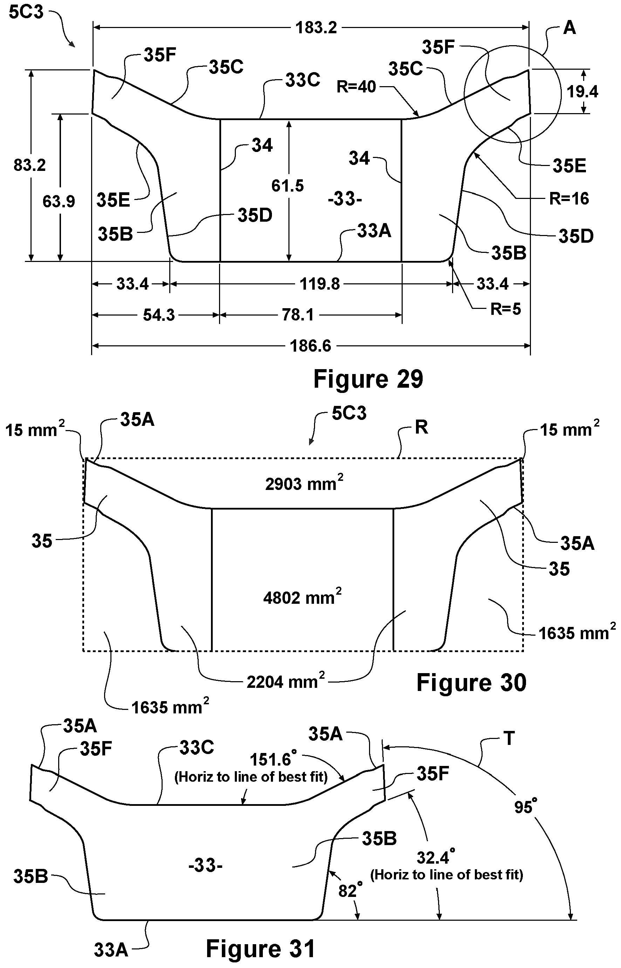

Referring to Figure 29, example relative dimensions (mm) of the rear panel 5C3 are:

- The main portion 33 is defined by a rectangle with dimensions of less than 100mm by 70mm, and in one example 78.1mm by 63.9mm.
- The lateral portions 35 are constrained within a rectangle of less than 60mm by 90mm, and in one example 54.3mm by 83.2 mm.
- The entire rear panel 5C is constrained within a rectangle R, shown in dashed line, of less than 200mm by 100mm, and in one example 186.6mm by 83.2mm.
- The ratio of the area of the lateral portions 35 to the area of the main portion 33 is between 0.8 and 0.9 and preferably 0.92.

As with rear panels 5C and 5C2, each lateral portion 35 can be considered to comprise a stem portion 35B which extends from main body portion 33 and is defined between the main body portion 33 and a lower side margin comprising lower and upper parts 35D, 35E. Each lateral portion 35 further comprises a projecting portion 35F which projects radially outwardly from the stem portion 35B and is defined by the upper part 35E of lower side margin, the tip 35A, and an upper margin 35C. Each of margins 35C, 35D, 35E are substantially straight in these examples, although one or more of them could be arcuate. The upper part 35D of lower side margin and the upper margin 35C may be substantially parallel, or they may be inclined toward or away from one another, towards tip 35A. Thus the projecting portion 35F may taper or flare along its length, or be of substantially constant width.

In respect of panel 5C3, the stem portion 35B is wider than that of panels 5C, 5C2, and is approximately 20% wider in this example. The lower part of each lateral arm 35 is therefore more oblong than panels 5C, 5C2, and inclines upwardly away from the base margin of main portion 33 at a steeper angle such that the stem part of each lateral arm 35 initially does not project outwardly as far as arms 35 of panels 5C, 5C2. For panels 5C, 5C2, the ratio of the width of each lateral arm 35 to the width of the main body portion 33 is between 0.4:1 and 0.5:1. For panel 5C3, the ratio of the width of each lateral arm 35 to the width of the main body portion 33 is between 0.6:1 and 0.8:1. These calculations are based on each lateral arm 35 terminating where the upper arm margin 35F joins the straight, horizontal part of the upper margin 33C of main body portion 33. This termination point is defined by a vertical plane 34 which extends through the intersection of upper arm margin 35C with the upper margin 33C of main body portion 33, as can best be seen in figures 9, 24 and 29.

Referring to Figure 30 the rear panel 5C3 can be effectively described with various example ratios relating the lateral portions 35 and main portion 33. Example ratios are listed below.

- The ratio of the width of a lateral portion 35 to the width of the main portion 33 may be between 0.7 and 0.8, and in one example is 0.69.
- The ratio of the height of a lateral portion 35 to the height of the main portion 33 may be between 1.2 and 1.5, and in one example is 1.30.
- The ratio of the width of the entire rear panel 5C3 to the width of the main portion 33 may be between 2.0 and 3.0, and in one example is 2.39.
- The ratio of the area of a lateral portion 35 to the area of the main portion 33 is may be between 0.4 and 0.5, and in one example is 0.46.
- The ratio of the area of both lateral portions 35 to the area of the entire rear panel 5C may be between 0.3 and 0.7, and in one example is 0.48.
- The ratio of a cut-out formed in the top of the rear panel 5C3 defined by the area A between the upper margin 41 of the rear panel 5C3 and a horizontal line 43 connecting the two most upper points 45 of the lateral portions 35 to the area of the entire rear panel 5C3 may be between 0.3 and 0.5, and in one example is 0.32.
- The rear panel 5C3 may be between 30 and 70%, and in one example makes up 59.32%, of a notional rectangular area A that extends to touch all extreme points of the rear panel 5C.
- The ratio of the width of the lower margin of the rear panel 5C3 to the width of the entire rear panel 5C3 may be between 0.4 and 0.8, and in one example is 0.64.

Referring to Figure 31, example dimensions of, and example angles between, the different parts of the rear panel 5C3 are shown. These include a lateral portion 35 tip angle T which defines the angle of the straight, distal end margin 35A of each lateral portion 35 relative to the horizontal base margin 33A of the main portion 33.

The angle between the lower part of the lower side margin of each lateral portion 35 and the base margin 33A of main portion 33 may be between 50 and 90°, for panel 5C and 5C2 is 70°, and for panel 5C3 is 82°. The lower part of the lower side margin is the part which extends from the base margin 33A of main portion 33. The angle between the upper part 35D of lower side margin (and the longitudinal axis of the projecting arm portion) of each lateral portion 35 and the base margin 33A of main portion 33 may be between 25 and 70°, for panel 5C is 45.5°, and for panel 5C3 is 32.4°.

The angle between the upper side margin 35D of each lateral portion 35 and the base margin 33A of main portion 33 may be between 100 and 180°, for panel 5C and 5C2 is 154.5°, and for panel 5C3 is 151.6°. The upper side margin 35D is the part which extends from the top margin 33C of main portion 33 to the tip 35A of each lateral portion 35.

The end margin of the tip 35A of each lateral portion 35 is preferably substantially straight. The angle between the end margin of the tip 35A of each lateral portion 35, and the base margin 33A of main portion 33 is preferably between 80 and 120°, and for panels 5C, 5C2 and 5C3 is 95°.

Unless the context clearly requires otherwise, throughout the description, the words "comprise", "comprising", and the like, are to be construed in an inclusive sense as opposed to an exclusive or exhaustive sense, that is to say, in the sense of "including, but not limited to".

The scope of the invention is defined by the appended claims.

Any discussion of the prior art throughout the specification should in no way be considered as an admission that such prior art is widely known or forms part of common general knowledge in the field.

## Claims

1. Headgear (5) configured to be secured to a patient interface (3) of a respiratory therapy system, to mount the patient interface (3) on a user's head, the headgear (5) comprising:

   a pair of side straps (5A), each side strap having first and second opposed ends, a first end of each strap being secured to, or being configured to be secured to, the patient interface (3) and to extend along the sides of the user's head;
   a rear panel (5C, 5C2) configured to engage the rear of the user's head, the second end of each side strap (5A) being joined to the rear panel (5C, 5C2) such that the pair of side straps (5A) extend from the rear panel (5C, 5C2); wherein
   the rear panel (5C, 5C2) comprises a main body (33) and a pair of laterally extending arms (35), the second end of each side strap being joined to a respective arm (35);
   the main body (33) comprising:

   　　opposed top and base margins (33C, 33A) which are spaced apart by a distance,
   　　substantially straight, outwardly inclined, opposed side margins (33B) extending between the top and base margins (33C, 33A), and
   　　a central longitudinal axis bisecting the top and base margins,
   　　wherein the area of the main body (33) is defined by the top and base margins (33C, 33A) and the side margins (33B), including the area defined where the top margin (33C) notionally extends towards the laterally extending arms (35) and intersects with where the side margins (33B) notionally extend towards the laterally extending arms (35);

   each laterally extending arm (35) being inclined relative to the central longitudinal axis, extending away from a respective side margin (33B) of the main body (33) and comprising a distal arm end (35A), each distal arm end (35A) having a width measured in a direction substantially parallel with the central longitudinal axis;
   wherein the ratio of the area of both lateral extending arms (35) to the main body (33) is between 0.1 and 0.3.

2. The headgear (5) of claim 1, wherein the total area of the rear panel (5C, 5C2) is between 6000 and 13000mm$^2$.

3. The headgear (5) of claim 1 or claim 2, wherein the ratio of the area of the main body (33) to the total area of the rear panel (5C, 5C2) is between 0.7 and less than 1.0.

4. The headgear (5) of any one of the preceding claims, wherein the laterally extending arms (35) intersect the main body (33) above a vertical midpoint of the main body (33).

5. The headgear (5) of any one of the preceding claims, wherein when the length of the top margin (33C) of the main body (33) divided into two lateral thirds and a central third, each laterally extending arm (35) can be said to intersect the top margin (33C) of the main body (33) within the lateral third proximal to the laterally extending arm (35) in question.

6. The headgear (5) of any one of the preceding claims, wherein the entire rear panel (5C, 52C) is constrained within a rectangle R of less than 200mm by 100mm.

7. The headgear (5) of any one of the preceding claims, wherein the side margins (33B) of the main body (33) of the rear panel (5C, 52C) are substantially equal length and are inclined relative to the base margin (33A) such that the main (33) body is substantially trapezoidal.

8. The headgear (5) of any one of the preceding claims, wherein two pairs of side straps (5A, 5B) are provided, one pair comprising upper side straps (5A), the other pair comprising lower side straps (5B).

9. The headgear (5) of any one of the preceding claims, wherein the distal arm end (35A) comprises a substantially straight end margin, wherein the end margin is inclined relative to the longitudinal axis and wherein, preferably, the end margin is inclined relative to the longitudinal axis at an angle between 0.1 and 20°.

10. The headgear (5) of any one of the preceding claims, wherein the side margins (33B) of the rear panel (33) are inclined relative to the longitudinal axis at a different angle to the angle of inclination of the laterally extending arms (35) relative to the longitudinal axis.

11. The headgear (5) of any one of the preceding claims, wherein the side margins (33B) of the rear panel (33) are inclined at an angle between 0° and 40° relative to the longitudinal axis of the rear panel (33).

12. The headgear (5) of any one of the preceding claims, wherein the longitudinal axis of each laterally extending arm (35) is inclined at an angle between 40° and 90° relative to the longitudinal axis of the rear panel (5C, 52C).

13. The headgear (5) of any one of the preceding claims, wherein each laterally extending arm (35) extends from a respective side margin (33B) and the top margin (33A) of the main body (33).

14. The headgear (5) of any one of the preceding claims, wherein the distal arm end (35A) of each laterally extending arm (35) is stepped or tapered or waisted, so that the distal arm end (35A) is of narrower width than the remainder of the laterally extending arm (35).

15. A patient interface assembly comprising;

> a patient interface (3), and
> the headgear (5) of any one of the preceding claims.

**Patentansprüche**

1. Kopfbedeckung (5), die so konfiguriert ist, dass sie an einer Patientenschnittstelle (3) eines Atemtherapiesystems gesichert werden kann, um die Patientenschnittstelle (3) am Kopf eines Benutzers anzubringen, wobei die Kopfbedeckung (5) umfasst:

> ein Paar Seitengurte (5A), wobei jeder Seitengurt ein erstes und ein zweites gegenüberliegendes Ende aufweist, wobei ein erstes Ende jedes Gurtes an der Patientenschnittstelle (3) gesichert ist oder so konfiguriert ist, dass es an dieser gesichert werden kann, und sich entlang der Seiten des Kopfes des Benutzers erstreckt;
> eine hintere Platte (5C, 5C2), die so konfiguriert ist, dass sie die Hinterseite des Kopfes des Benutzers in Eingriff nimmt, wobei das zweite Ende jedes Seitengurts (5A) so mit der hinteren Platte (5C, 5C2) zusammengefügt ist, dass sich das Paar Seitengurte (5A) von der hinteren Platte (5C, 5C2) erstreckt; wobei
> die hintere Platte (5C, 5C2) einen Hauptkörper (33) und ein Paar sich seitlich erstreckender Arme (35) umfasst, wobei das zweite Ende jedes Seitengurts mit einem jeweiligen Arm (35) zusammengefügt ist;
> der Hauptkörper (33) umfassend:
>
>> einen Oberrand und einen gegenüberliegenden Basisrand (33C, 33A), die um einen Abstand voneinander beabstandet sind,

im Wesentlichen gerade, nach außen geneigte, gegenüberliegende Seitenränder (33B), die sich zwischen dem Oberrand und Basisrand (33C, 33A) erstrecken, und

eine Mittellängsachse, die den Oberrand und den Basisrand halbiert;

wobei die Fläche des Hauptkörpers (33) durch den Oberrand und den Basisrand (33C, 33A) und die Seitenränder (33B) definiert ist, einschließlich der Fläche, die dort definiert ist, wo sich der Oberrand (33C) theoretisch in Richtung der sich seitlich erstreckenden Arme (35) erstreckt und sich dort mit diesen schneiden, wo sich die Seitenränder (33B) theoretisch in Richtung der seitlich erstreckenden Arme (35) erstrecken;

wobei jeder sich seitlich erstreckende Arm (35) relativ zu der Mittellängsachse geneigt ist, sich von einem jeweiligen Seitenrand (33B) des Hauptkörpers (33) weg erstreckt und ein distales Armende (35A) umfasst, wobei jedes distale Armende (35A) eine in einer im Wesentlichen zur Mittellängsachse parallelen Richtung gemessene Breite aufweist;

wobei das Verhältnis der Fläche beider sich seitlich erstreckender Arme (35) zum Hauptkörper (33) zwischen 0,1 und 0,3 liegt.

2. Kopfbedeckung (5) nach Anspruch 1, wobei die Gesamtfläche der hinteren Platte (5C, 5C2) zwischen 6.000 und 13.000 mm$^2$ beträgt.

3. Kopfbedeckung (5) nach Anspruch 1 oder Anspruch 2, wobei das Verhältnis der Fläche des Hauptkörpers (33) zur gesamten Fläche der hinteren Platte (5C, 5C2) zwischen 0,7 und weniger als 1,0 liegt.

4. Kopfbedeckung (5) nach einem der vorstehenden Ansprüche, wobei die sich seitlich erstreckenden Arme (35) den Hauptkörper (33) oberhalb eines vertikalen Mittelpunkts des Hauptkörpers (33) schneiden.

5. Kopfbedeckung (5) nach einem der vorstehenden Ansprüche, wobei, wenn die Länge des Oberrands (33C) des Hauptkörpers (33) in zwei seitliche Drittel und ein mittleres Drittel unterteilt ist, jeder sich seitlich erstreckende Arm (35) den Oberrand (33C) des Hauptkörpers (33) innerhalb des seitlichen Drittels proximal des betreffenden sich seitlich erstreckenden Arms (35) schneidet.

6. Kopfbedeckung (5) nach einem der vorstehenden Ansprüche, wobei die gesamte hintere Platte (5C, 52C) innerhalb eines Rechtecks R von weniger als 200 mm x 100 mm eingezwängt ist.

7. Kopfbedeckung (5) nach einem der vorstehenden Ansprüche, wobei die Seitenränder (33B) des Hauptkörpers (33) der hinteren Platte (5C, 52C) im Wesentlichen gleich lang sind und relativ zum Basisrand (33A) so geneigt sind, dass der Hauptkörper (33) im Wesentlichen trapezförmig ist.

8. Kopfbedeckung (5) nach einem der vorstehenden Ansprüche, wobei zwei Paare an Seitengurten (5A, 5B) bereitgestellt sind, wobei ein Paar obere Seitengurte (5A) umfasst und das andere Paar untere Seitengurte (5B) umfasst.

9. Kopfbedeckung (5) nach einem der vorstehenden Ansprüche, wobei das distale Armende (35A) einen im Wesentlichen geraden Endrand umfasst, wobei der Endrand relativ zur Längsachse geneigt ist und wobei der Endrand relativ zur Längsachse vorzugsweise in einem Winkel zwischen 0,1 und 20° geneigt ist.

10. Kopfbedeckung (5) nach einem der vorstehenden Ansprüche, wobei die Seitenränder (33B) der hinteren Platte (33) relativ zur Längsachse in einem Winkel geneigt sind, der sich vom Neigungswinkel der sich seitlichen erstreckenden Arme (35) relativ zur Längsachse unterscheidet.

11. Kopfbedeckung (5) nach einem der vorstehenden Ansprüche, wobei die Seitenränder (33B) der hinteren Platte (33) in einem Winkel zwischen 0° und 40° relativ zur Längsachse der hinteren Platte (33) geneigt sind.

12. Kopfbedeckung (5) nach einem der vorstehenden Ansprüche, wobei die Längsachse jedes sich seitlich erstreckenden Arms (35) in einem Winkel zwischen 40° und 90° relativ zur Längsachse der hinteren Platte (5C, 52C) geneigt ist.

13. Kopfbedeckung (5) nach einem der vorstehenden Ansprüche, wobei jeder sich seitlich erstreckende Arm (35) sich von einem jeweiligen Seitenrand (33B) und dem Oberrand (33A) des Hauptkörpers (33) erstreckt.

**14.** Kopfbedeckung (5) nach einem der vorstehenden Ansprüche, wobei das distale Armende (35A) jedes sich seitlich erstreckenden Arms (35) abgestuft oder verjüngt oder tailliert ist, sodass das distale Armende (35A) eine geringere Breite als der Rest des sich seitlich erstreckenden Arms (35) hat.

**15.** Patientenschnittstellenanordnung, umfassend

eine Patientenschnittstelle (3), und
die Kopfbedeckung (5) nach einem der vorstehenden Ansprüche.

**Revendications**

**1.** Casque (5) configuré pour être fixé à une interface patient (3) d'un système de thérapie respiratoire, pour monter l'interface patient (3) sur la tête d'un utilisateur, le casque (5) comprenant :

une paire de sangles latérales (5A), chaque sangle latérale présentant des première et seconde extrémités opposées, une première extrémité de chaque sangle étant fixée à, ou étant configurée pour être fixée à l'interface patient (3) et pour s'étendre le long des côtés de la tête de l'utilisateur ;
un panneau arrière (5C, 5C2) configuré pour venir en prise avec l'arrière de la tête de l'utilisateur, la seconde extrémité de chaque sangle latérale (5A) étant reliée au panneau arrière (5C, 5C2) de telle sorte que la paire de sangles latérales (5A) s'étend depuis le panneau arrière (5C, 5C2) ; dans lequel
le panneau arrière (5C, 5C2) comprend un corps principal (33) et une paire de bras (35) s'étendant latéralement, la deuxième extrémité de chaque sangle latérale étant reliée à un bras (35) respectif ;
le corps principal (33) comprenant :

des bordures supérieure et de base opposées (33C, 33A) qui sont espacées d'une certaine distance, des bordures latérales opposées sensiblement droites, inclinées vers l'extérieur (33B), s'étendant entre les bordures supérieure et de base (33C, 33A), et
un axe longitudinal central coupant en deux les bordures supérieure et de base,
dans lequel la surface du corps principal (33) est délimitée par les bordures supérieure et de base (33C, 33A) et les bordures latérales (33B), incluant la surface délimitée à l'endroit où la bordure supérieure (33C) s'étend théoriquement vers les bras (35) s'étendant latéralement et croise l'endroit où les bordures latérales (33B) s'étendent théoriquement vers les bras (35) s'étendant latéralement ;

chaque bras s'étendant latéralement (35) étant incliné par rapport à l'axe longitudinal central, s'étendant à l'opposé d'une bordure latérale respective (33B) du corps principal (33) et comprenant une extrémité de bras distale (35A), chaque extrémité de bras distale (35A) présentant une largeur mesurée dans une direction sensiblement parallèle à l'axe longitudinal central ;
dans lequel le rapport entre la surface des deux bras s'étendant latéralement (35) et le corps principal (33) est compris entre 0,1 et 0,3.

**2.** Casque (5) selon la revendication 1, dans lequel la surface totale du panneau arrière (5C, 5C2) est comprise entre 6000 et 13000 mm$^2$.

**3.** Casque (5) selon la revendication 1 ou la revendication 2, dans lequel le rapport entre la surface du corps principal (33) et la surface totale du panneau arrière (5C, 5C2) est compris entre 0,7 et moins de 1,0.

**4.** Casque (5) selon l'une quelconque des revendications précédentes, dans lequel les bras s'étendant latéralement (35) croisent le corps principal (33) au-dessus d'un point médian vertical du corps principal (33).

**5.** Casque (5) selon l'une quelconque des revendications précédentes, dans lequel, lorsque la longueur de la bordure supérieure (33C) du corps principal (33) est divisée en deux tiers latéraux et un tiers central, chaque bras s'étendant latéralement (35) peut être considéré comme coupant la bordure supérieure (33C) du corps principal (33) à l'intérieur du tiers latéral proximal par rapport au bras s'étendant latéralement (35) en question.

**6.** Casque (5) selon l'une quelconque des revendications précédentes, dans lequel l'ensemble du panneau arrière (5C, 52C) s'inscrit dans un rectangle R inférieur à 200 mm par 100 mm.

7. Casque (5) selon l'une quelconque des revendications précédentes, dans lequel les bordures latérales (33B) du corps principal (33) du panneau arrière (5C, 52C) sont de longueur sensiblement égale et sont inclinées par rapport à la bordure de base (33A) de sorte que le corps principal (33) est sensiblement trapézoïdal.

8. Casque (5) selon l'une quelconque des revendications précédentes, dans lequel deux paires de sangles latérales (5A, 5B) sont prévues, une paire comprenant des sangles latérales supérieures (5A), l'autre paire comprenant des sangles latérales inférieures (5B).

9. Casque (5) selon l'une quelconque des revendications précédentes, dans lequel l'extrémité de bras distale (35A) comprend une bordure d'extrémité sensiblement droite, dans lequel la bordure d'extrémité est inclinée par rapport à l'axe longitudinal et dans lequel, de préférence, la bordure d'extrémité est inclinée par rapport à l'axe longitudinal selon un angle compris entre 0,1 et 20°.

10. Casque (5) selon l'une quelconque des revendications précédentes, dans lequel les bordures latérales (33B) du panneau arrière (33) sont inclinées par rapport à l'axe longitudinal selon un angle différent de l'angle d'inclinaison des bras (35) s'étendant latéralement par rapport à l'axe longitudinal.

11. Casque (5) selon l'une quelconque des revendications précédentes, dans lequel les bordures latérales (33B) du panneau arrière (33) sont inclinées selon un angle compris entre 0° et 40° par rapport à l'axe longitudinal du panneau arrière (33).

12. Casque (5) selon l'une quelconque des revendications précédentes, dans lequel l'axe longitudinal de chaque bras (35) s'étendant latéralement est incliné selon un angle compris entre 40° et 90° par rapport à l'axe longitudinal du panneau arrière (5C, 52C).

13. Casque (5) selon l'une quelconque des revendications précédentes, dans lequel chaque bras s'étendant latéralement (35) s'étend depuis une bordure latérale respective (33B) et depuis la bordure supérieure (33A) du corps principal (33).

14. Casque (5) selon l'une quelconque des revendications précédentes, dans lequel l'extrémité de bras distale (35A) de chaque bras s'étendant latéralement (35) est étagée ou effilée ou cintrée, de sorte que l'extrémité de bras distale (35A) est de largeur plus étroite que le reste du bras s'étendant latéralement (35).

15. Ensemble interface patient comprenant ;

une interface patient (3), et
le casque (5) selon l'une quelconque des revendications précédentes.

**Figure 1**

**Figure 2**

**Figure 3**

**Figure 4**

Figure 5

Figure 6

**Figure 7**

**Figure 8**

90EP 4 420 703 B1

**Figure 9**

**Figure 10**

9020

**Figure 11**

**Figure 12**

**Figure 13**

**Figure 14**

**Figure 15**

**Figure 16**

35B  35C
y  Main Portion
(trapezoid)
35A
Lateral Portion 35
33B  70°
R=10 fillet  33A

x
33C
35C  35B
y
35A
35 Lateral Portion
33B
-33-
x
R=10 fillet

**Figure 17**

5°
35C
154.5°
y
-35-
35B
35A  153.9°  y
33B
70°
33A

R=40 fillet
x
33C
35C  35B
y
-35-  35A
-33-
33B
R=10 fillet
x

**Figure 18**

Figure 19

Figure 20

**Figure 21**

**Figure 22**

5C2

24 mm$^2$

R

24 mm$^2$

35A

35A

2825 mm$^2$

2110 mm$^2$

2110 mm$^2$

35

35

1866 mm$^2$

4789 mm$^2$

1866 mm$^2$

33

**Figure 23**

5C2

A

35A

186.5

35F

35F

35C

35C

35

35

35

24.7

33C

82.5

34

34

61.5

57.9

35A

35E

-33-

35E

35B

35D

33A

35D

46.6

97.7

46.6

35B

56.5

77.9

R=5

190.8

**Figure 24**

35A

35A

T

35F

33C

154.2°
(Horiz to line of best fit)

35

35F

95°

-33-

35

25.5°
(Horiz to line of best fit)

70°

33A

**Figure 25**

35B

**Figure 26**

**Figure 27**

**Figure 28**

**Figure 29**

**Figure 30**

**Figure 31**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012145157 A1